# EUROPEAN PATENT SPECIFICATION

(11) **EP 2 635 289 B1**
(45) Date of publication and mention of the grant of the patent: **11.03.2020**
(21) Application number: 11785050.3
(22) Date of filing: 04.11.2011
(51) Int. Cl.: A61K 31/765, A61P 35/00

(54) **PEG OR PEG BLOCK COPOLYMERS FOR TREATING COLORECTAL CANCER**
PEG ODER PEG BLOCK COPOLYMERS ZUR BEHANDLUNG VON KOLOREKTALKREBS
PEG OU COPOLYMÈRES DU PEG POUR LE TRAITEMENT DU CANCER COLORECTAL

(30) Priority: 10.11.2010 US 412128 P; 04.11.2010 GB 201018650
(43) Date of publication of application: 11.09.2013
(73) Proprietor: Norgine B.V., 1101 CA Amsterdam ZO (NL)
(72) Inventor: STEIN, Peter, Uxbridge, Middlessex UB9 6NS (GB); COX, Ian, Hengoed, Mid-Glamorgan CF82 8SJ (GB); SMITH, Samuel, Hengoed, Mid-Glamorgan CF82 8SJ (GB); JONES, Leighton, Hengoed, Mid-Glamorgan CF82 8SJ (GB); PLESSL, Jörg, Hengoed, Mid-Glamorgan CF82 8SJ (GB); DE VRIES, Corinne, Hengoed, Mid-Glamorgan CF82 8SJ (GB); CHARLTON, Rachel, Hengoed, Mid-Glamorgan CF82 8SJ (GB)
(74) Representative: Abel & Imray
(86) International application number: PCT/GB2011/001561
(87) International publication number: WO 2012/059725

(56) References cited:
- US-A1- 2001 051 659

## Description

The present invention concerns compositions for use in methods for the prevention of colorectal cancer (CRC) in humans.

Colorectal cancer is a major cause of death in the human population, particularly in North America and Europe. Prevention is urgently required and therefore research into various strategies including control of diet and other lifestyle modifications, including chemical interventions, has been reported.

In International Patent Application No: WO00/24407, the use of a non-fermented osmotic laxative such as polyethylene glycol (PEG) to treat and prevent colorectal cancer is disclosed. This finding was based on work carried out in an azoxymethane (AOM) model of colon cancer in Fischer F344 rats. It suggests that the doses used in rats may be translated into a daily dose in humans of between 10 to 80g. However, no direct evidence is disclosed that a therapeutic effect for PEG exists in human forms of CRC nor over what timeframe such an exposure is necessary to achieve such an effect.

Dorval E. et al; Gastro Clin Biol, 30:1196-1199 (2006), investigated, in a human population based study, the prevalence of colorectal adenomas in association with dietary PEG consumption. Consecutive patients, attending hospital for routine total colonoscopy, were asked if they had previously taken a laxative or a non-steroidal antiinflammatory drug (NSAID). Based on the answers provided, the authors concluded that use of Forlax®, a PEG containing bowel preparation, was associated with a lower incidence of colorectal tumours, yet other PEG or PEG-like laxatives including MOVICOL®, Transipeg® and Idrocol® did not produce significant results. The authors posited that one reason may have been that the "PEG dose varies from one brand to another, and appears to be among the highest in Forlax®. As noted in the paper, since the study was based on a patient based questionnaire, "this survey did not yield reliable information regarding the duration, quantity, regularity or timing of PEG ingestion."

In addition to the above-noted references, there have been other studies involving rodents building on the work mentioned above, which generally tend to the view that a relatively high dose of a PEG in a relatively short time frame can reduce incidences of ACF and EGFR - believed to be markers for colon cancer. There is no apparent consensus regarding the timing of the administration of the PEG in order to be effective in reduction of the surrogate marker(s) and the level of dose of the PEG, if scaled up for human use, would result in diarrhoea. The requirement for a high dose is echoed in the Dorval (2006) reference, which shows, at table II, that MOVICOL® (a PEG plus electrolytes composition) is ineffective; similarly, it shows that the combined results of PEG-containing laxatives are not significant. To date, this appears to be the only published study related to the human use of PEG in the context of colorectal cancer, yet it does not exclude data from patients whose constipation (and the reason for them taking a laxative such as Forlax® or MOVICOL®) is caused by, or is a symptom of, colorectal cancer.

Surprisingly, when the present inventors instigated a study of a General Practice Research Database (GPRD) to describe the incidences of colorectal cancer in the human population following different types of laxative exposure by comparison with controls sampled from the same laxative cohort population but having no diagnosis of colorectal cancer, they found a potential dose-response relationship in patients ingesting over 60 sachets of MOVICOL®, both 24 and 36 months prior to the earliest date they presented with evidence of a CRC diagnosis. MOVICOL is provided in a sachet containing 13.8g powder for making up into an oral solution. Each sachet contains: 13.1250g Macrogol (polyethylene glycol (PEG)) 3350, 0.3507g sodium chloride, 0.1785g sodium bicarbonate and 0.0466g potassium chloride. This is the standard dose of MOVICOL. It also contains flavouring and sweetener. MOVICOL has been on the market since 1995.

Therefore, in accordance with the present invention there is provided a composition for use in a method for preventing CRC in a human comprising administering between 800 grams and 2365 grams of PEG over a period of 18 to 36 consecutive calendar months.

Also disclosed herein is a method of treating, ameliorating and/or preventing colorectal cancer (CRC) in a human which method comprises administering to the human an effective and sub-laxative dose of PEG.

Also disclosed herein is a method for treating, ameliorating and/or preventing CRC in a human which method comprises administering to the human an effective amount and sub-laxative dose of PEG.

Also disclosed herein is a method; (a) for preventing constipation and; (b) of preventing CRC in a human which method comprises administering an effective and sub-laxative dose of PEG.

Also disclosed herein is a method; (a) for preventing constipation and; (b) for preventing CRC in a human which method comprises administering an effective and sub-laxative dose of PEG.

Also disclosed herein is a method; (a) for preventing constipation and; (b) for ameliorating CRC in a human which method comprises administering to the human an effective and sub-laxative dose of PEG.

Also disclosed herein is a method; (a) for preventing constipation and; (b) for treating CRC in a human which method comprises administering to the human an effective and sub-laxative dose of PEG

Also disclosed herein is a composition suitable for human administration comprising, as a unit dose, a sub-laxative amount of PEG.

Also disclosed herein is a composition for use in a method of the invention described *supra* comprising, as a unit dose, a sub-laxative amount of PEG.

The term "sub-laxative amount" means an amount that does not increase the average number of stools per week for the human subject concerned.

The term "sub-laxative dose", means a dose that does not increase the average number of stools per week for the human subject concerned.

The term "PEG" means polyethylene glycol having the general formula H-(O-CH₂-CH₂)ₙ -OH.

The term "PEG block co-polymer" refers to a co-polymer of polyethylene glycol with polypropylene glycol, namely, polyethylene polypropylene glycol. Exemplary PEG block co-polymers include those available under the tradename "Pluronic® F68" or "poloxamer 188".

The term "treating" and grammatical variations thereof is intended to mean that the compositions for use as described herein may be able to cure and/or reverse the normal disease course of CRC, particularly early stage colorectal pathologies associated with the development of CRC, for example by treating colon polyps.

The term "ameliorating" and grammatical variations thereof is intended to mean that the compositions for use as described herein can slow or stop the progression of early stage colorectal pathologies towards the development of CRC, e.g. slow or stop the further development of colon polyps and/or aberrant crypt foci (ACF) into CRC.

The term "preventing" and grammatical variations thereof is intended to mean that the compositions for use as described herein reduce the risk of developing CRC and/or (as the case may be) constipation.

The term "colorectal cancer" or "CRC" refers to colorectal, colon and/or rectal cancer in humans.

The term "one month" means a contiguous 30 day period.

The phrase "a method for" is intended to denote a purposive method.

In certain embodiments of the invention described *supra,* a dose (e.g. an effective and sub-laxative dose) is administered to provide between 800 and 2365 (e.g. 800.6 to 2362.5) grams of PEGover a period of 36 consecutive calendar months.

In certain embodiments of the invention described *supra,* a dose (e.g. an effective and sub-laxative dose) is administered to provide between 800 and 2365 (e.g. 800.6 to 2362.5) grams of PEG over a period of 24 consecutive calendar months.

In certain embodiments of the invention described *supra,* a dose (e.g. an effective and sub-laxative dose) is administered to provide between 266 grams and 1181 grams (e.g. between 266.9 grams and 787.5 grams; between 400.3 grams and 1181.3 grams; between 400.3 grams and 787.5 grams) of PEG over a period of 12 consecutive calendar months.

In certain embodiments of the invention described *supra,* a dose (e.g. an effective and sub-laxative dose) is administered to provide between 22.2 grams and 98.4 grams (e.g. between 33.3 grams and 98.4 grams; 22.2 grams and 65.6 grams; 65.6 grams and 98.4 grams) of PEG over a period of one month.

In certain embodiments of the invention described *supra,* a dose (e.g. an effective and sub-laxative dose) is administered to provide between 5.1 grams and 22.7 grams (e.g. between 7.7 grams and 22.7 grams; 5.1 grams and 15.1 grams; 15.1 grams and 22.7 grams) of PEG over a period of one week.

In certain embodiments of the invention described *supra,* a dose (e.g. an effective and sub-laxative dose) is administered to provide between 0.73 grams and 3.22 grams (e.g. between 1.1 grams and 3.2 grams; 0.73 grams and 2.2 grams; 2.2 grams to 3.2 grams) of PEG over a period of one day. Such doses may be administered daily over period of 24 or 36 consecutive calendar months. Alternatively, such doses may be administered intermittently over a period of 24 or 36 consecutive calendar months.

In all embodiments described herein, it will be apparent to the reader of this specification that ranges specified as being "between" or "within" two values are to be understood as being inclusive of those values, for example "between 1.1 grams and 3.2 grams" includes 1.1 grams and 3.2 grams respectively.

In other embodiments of the invention, compositions for use in a method (such as the methods described *supra*) for preventing CRC in humans comprising an amount (e.g. effective and sub-laxative) of PEG are provided.

In certain embodiments, the composition is in the form of a tablet (e.g. chewable or suckable tablet), capsule, caplet, troche, liquid, powder (e.g. powder for solution or suspension) and granules. Preferably, it is the form of a solid composition for oral administration. It may be a solid tablet, for example a chewable and/or suckable tablet.

In some embodiments of the invention, the PEG has an average molecular weight of at least 400, or at least 1000, or between 2000 to 10000 Daltons (e.g. 2500 to 8500, preferably 3000 to 8000 (e.g. 6000 or 8000), more preferably 2500 to 4500, e.g. 3350 or 4000). In certain embodiments, a blend of PEG molecules is provided of differing molecular weights to give a desired overall average molecular weight (for example an average molecular weight of 3350 or thereabout, or an average molecular weight of 4000 or thereabout). Exemplary PEG products are macrogol 3350, macrogol 4000, macrogol 6000, macrogol 8000 and are available commercially.

Also disclosed herein is a solid composition for oral administration as a solid for treating, ameliorating and/or preventing CRC comprising:
(a) 50 - 90 % w/w polyethylene glycol (PEG) having an average molecular weight within the range 2,000 to 10,000 Da; and
(b) 10 - 40 % w/w of a solid (sometimes referred to herein as "solid of component (b)")

Also disclosed herein is a solid composition for oral administration as a solid for treating, ameliorating and/or preventing CRC comprising:
(a) 50 - 90 % w/w polyethylene glycol (PEG) having an average molecular weight within the range 2,000 to 10,000 Da;
(b) 10 - 40 % w/w of a solid; and optionally
(c) q.s. to 100% w/w of further excipients such as flavourings, sweeteners and lubricants.

Herein, "% w/w" of a component is understood to mean the proportion, as a percentage, that the weight of the respective component makes up of the total weight of the solid composition.

The present invention further provides a composition for use in a method for preventing CRC in humans as described *supra,* wherein the composition comprises:
(a) 50 - 90 % w/w polyethylene glycol (PEG) having an average molecular weight within the range 2,000 to 10,000 Da; and
(b) 10 - 40 % w/w of a solid selected from the group: sorbitol, lactose, lactose and starch (e.g. a compound comprising lactose monohydrate and maize starch such as Starlac®), dextrates, cellulose (such as microcrystalline cellulose), xylitol, maltitol and mannitol;
(c) optionally further excipients such as flavourings, sweeteners and lubricants.

Preferably, the solid of component (b) is greater than 10%w/w, preferably greater than 12% w/w, more preferably greater than 15% (e.g. 15% to 17%) of the solid composition. Preferably the weight ratio of component (a) to component (b) is 1.25:1 to 9:1, preferably 2:1 to 7:1, preferably 4:1 to 6:1. In preferred embodiments, the ratio of component (a) to component (b) is approximately 5:1.

Preferably, the solid composition is chewable and/or suckable. It may be a solid tablet, for example a chewable and/or suckable tablet.

It has surprisingly been found that a solid composition for use in the invention as described *supra* comprising PEG component (a) and solid component (b) is pleasantly chewable or suckable, has good taste, structural integrity and beneficial manufacturing properties. By "chewable" or "suckable" is meant herein that the solid composition is for oral administration and is capable of being chewed or sucked in the mouth so that the first step in the digestive process starts in the buccal cavity.

Compositions for use in the invention may further comprise electrolytes. Compositions for use in the invention are preferably substantially free from electrolytes.

For example, they are preferably substantially free from sodium chloride, potassium chloride and sodium bicarbonate. They are preferably substantially free from sulphates or phosphates, for example, they are particularly preferred to be substantially free from sodium sulphate. Compositions for use in the invention are preferably substantially free from carbonates, bicarbonates, alkali metal ions and halide ions. Compositions for use in the present invention are most preferably substantially free from sodium, potassium, chloride, bicarbonate, carbonate and/or sulphate ions. In many instances, flavourings, lubricants and sweeteners may contain small amounts of electrolytes. Such amounts are not considered herein to be "substantial". Compositions for use in the invention are preferably substantially free from alginates and/or ascorbates and/or citrates. By "substantially free from" herein is also meant that the ingredient is not added to the composition during preparation or manufacture.

In some embodiments, compositions for use in the present invention are substantially free from any osmotic agent other than PEG.

The polyethylene glycol (PEG) preferably has an average molecular weight (for example a weight average molecular weight), in Daltons, within the range 2,000 to 10,000, preferably 2,500 to 8,500, preferably 3,000 to 8,000, more preferably 3,000 to 6,000, more preferably 2,500 to 6,500, more preferably 2,500 to 4,500 for example 3,000 to 4,500, for example 3,000 to 4,100, for example 3,000 to 4,000. The PEG may have an average molecular weight within the range 6,000 to 10,000, for example 7,000 to 9,000. For example, the PEG may be, or comprise PEG 3,350, PEG 4,000 or PEG 8,000 as defined in national or regional pharmacopoeias. Further examples of suitable PEGs recognized in some national pharmacopoeias include Macrogols, for example Macrogol 4,000. Optionally, the PEG used in the compositions may comprise two or more different PEG components. Optionally, the PEG used in compositions may have at least two differing average molecular weights. PEG of the relevant molecular weights in a form suitable for use in humans is available commercially.

In a preferred embodiment, PEG is present in the solid composition in an amount of 60 to 90% w/w, preferably 70 to 90% w/w, more preferably 70 to 89 % w/w, for example 75 to 89 % w/w. In a further embodiment, PEG is present in an amount of 78 to 89 % w/w, for example 80 to 85% w/w, for example 81 to 85 % w/w, for example 80 to 84 % w/w, for example 82 to 84 % w/w. In a further embodiment, PEG is present in an amount of 50 to 80% w/w, for example 60 to 80% w/w, for example 70 to 80 % w/w, for example 70 to 79% w/w, for example 75 to 79% w/w.

In some embodiments, the composition comprises between 0.1 to 6.5 grams (e.g. 0.73 grams to 3.2 grams; 1.1 grams to 3.2 grams; 0.73 grams to 2.2 grams) of PEG. In other embodiments, the composition comprises 5 grams or less of PEG. In other embodiments the composition comprises between 0.2 grams to 6.5 grams (e.g. 0.3 grams to 5.0 grams) of PEG. In further embodiments, the composition comprises between 1 to 4 grams of PEG. In other embodiments, the composition comprises between 1.5 and 2.5 grams of PEG. In other embodiments, the composition comprises between 2.0 grams to 2.5 grams of PEG, for example 2.2 grams or thereabout.

The solid of component (b) is selected from the group consisting of sorbitol, lactose, lactose and starch, dextrates, cellulose (e.g. microcrystalline cellulose), xylitol, maltitol, mannitol. Lactose or similar ingredients may be present in hydrated form.

Where the solid of component (b) is lactose and starch, the lactose component maybe in the form of a monohydrate. The starch component maybe derived from any suitable source such as wheat starch, maize starch, potato starch and rice starch. The lactose component may make up 50% to 95% of the lactose/starch solid, for example 60% to 90%, e.g. 70 to 85% such as 85%.

Solids of component (b) are preferably of a purity and grade suitable for consumption by e.g. humans.

The solid of component (b) makes up 10 to 40 % w/w of the solid composition. In a preferred embodiment, the solid of component (b) makes up 10 to 30% w/w of the solid composition, preferably greater than 10% w/w up to (and including) 30%w/w. For example, the solid of component (b) makes up 10 to 25% w/w, for example 10 to 20% w/w, preferably 12 to 20 % w/w, more preferably 12 to 19% w/w, 12 to 18% w/w, or 12 to 17 % w/w. For example, the solid of component (b) may make up 14 to 20% w/w, 14 to 19% w/w or 14 to 18% w/w 14 to 17% w/w of the solid composition such as 15 to 16.5%w/w of the solid composition.

Preferably, the solid of component (b) is mannitol. It has been found that a solid composition comprising PEG and mannitol is more palatable than a solid composition comprising PEG and no mannitol, even if flavouring is added. In particular, it has been found that a tablet comprising PEG and mannitol has a much lower requirement for lubricant or lubrication during tablet manufacture than a tablet comprising PEG but no mannitol. A high level of a lubricant in a tablet generally makes the tablet have an unacceptable taste. The reduced level (or absence of) a lubricant as compared with a tablet comprising PEG but no mannitol brings about an improved palatability (taste and mouthfeel) of a chewable or suckable tablet comprising PEG and mannitol. Such tablets are thus particularly suitable for use in the current invention.

Typically, solid compositions of dry ingredients are manufactured using dry granulation followed by punching with punch and die equipment. In a punch and die machine, dry ingredients are compressed together. It has surprisingly been found that a solid composition comprising PEG and mannitol in the specified proportions has better structural integrity and is more convenient to manufacture than a solid composition comprising PEG and no mannitol, or a smaller proportion of mannitol. Solid compositions of that type are less susceptible to capping and laminating during punch and die manufacture than solid compositions comprising a smaller proportion of mannitol, or no mannitol. Solid compositions that become capped or laminated during die pressing are not suitable for use and they become waste. It has been found that a solid composition containing between 50 and 90% w/w PEG and 10 to 40% w/w mannitol has better tablet pressing characteristics than a solid composition containing no mannitol or 10%w/w or less mannitol, for example less than 10% mannitol. Such compositions are thus particularly suitable for use in the current invention.

Mannitol may make up 10 to 40 % w/w of the solid composition. In a preferred embodiment, mannitol makes up 10 to 30% w/w of the solid composition. For example, mannitol makes up 10 to 25% w/w, for example 10 to 20% w/w, preferably 12 to 20 % w/w, more preferably 12 to 19% w/w, 12 to 18% w/w, or 12 to 17 % w/w. For example, mannitol may make up 14 to 20% w/w, 14 to 19% w/w or 14 to 18% w/w 14 to 17% w/w of the solid composition. Mannitol may be provided in various physical forms. For example, mannitol is available commercially in granular, powder or spray-dried form. In a preferred embodiment, the mannitol is granular. Mannitol is commercially available from several suppliers, including Merck, SPI Polyols Inc and Roquette.

In an embodiment, the PEG and mannitol are present in a weight ratio of PEG:mannitol of 1.25:1 to 9:1 (e.g. 3:1 to 9:1, or 4:1 to 9:1), preferably 2:1 to 7:1, preferably 4:1 to 6:1 or 4:1 to 8:1, for example 5:1 to 6:1. In preferred embodiments the ratio of PEG to mannitol 5:1 or thereabout.

The structural integrity of the solid composition is retained when the mannitol is granular mannitol. It is surprising that the solid composition is so structurally sound with granular mannitol. In general it is found that granular mannitol cannot be used with concentrations of other materials exceeding 25% by weight (Handbook of Pharmaceutical Excipients, 5th Ed., Pharmaceutical Press, 2006, page 452). It has been found that solid compositions for use in the invention, which comprise 60 to 90% w/w of materials other than mannitol, are readily manufacturable and have good structural integrity.

It has also surprisingly been found that a solid composition for use in the invention, comprising PEG and mannitol in the specified proportions is less prone to sticking to punch and die equipment than a solid composition comprising PEG and a smaller proportion of mannitol, or no mannitol. This is particularly important when manufacturing compositions for use in the present invention at commercial scale since fouling of the manufacturing machinery may lead to manufacturing down-time with the increased costs associated therewith.

Lubricants can be included in tablet compositions to reduce the propensity for them to stick to the punch or die after die pressing. Examples of lubricants include magnesium stearate, potassium stearate, talc, stearic acid, sodium lauryl sulphate, and paraffin. Mixtures of different lubricants may be used. It has been found that a solid composition for use in the invention, comprising PEG and mannitol in the specified proportions, requires a smaller proportion of lubricant to satisfactorily avoid sticking than a tablet comprising PEG and a smaller proportion of mannitol, or no mannitol. Preferably, a solid composition comprises lubricant in an amount of 2.0% w/w or less, for example 1.5% w/w or less, or 1.0% w/w or less. For example it may comprise lubricant in an amount of 0.1 to 0.9% w/w, for example 0.2 to 0.8% w/w, preferably 0.3 to 0.7% w/w. For example, the lubricant is present in ratio of solid of component (b) (such as mannitol): lubricant ratio of 170:1 to 16:1, for example 57:1 to 20:1. A particularly preferred lubricant is magnesium stearate. If the lubricant is magnesium stearate, it is effective to satisfactorily avoid sticking when used at a level of under 1% w/w. Accordingly, in an embodiment, the tablet of the invention further comprises magnesium stearate in an amount of 0.1 to 0.9% w/w, for example 0.2 to 0.8% w/w, preferably 0.3 to 0.7% w/w, more preferably 0.5% w/w. It is surprising that magnesium stearate is effective at these levels as, in general, magnesium stearate is required at a level of over 1% in compositions comprising mannitol (Handbook of Pharmaceutical Excipients, 5th Ed., Pharmaceutical Press, 2006, page 452).

In some embodiments, the composition comprises;
(a) 50 - 90 % w/w polyethylene glycol (PEG) having an average molecular weight within the range 2,000 to 10,000 Da;
(b) 10 - 40 % w/w of mannitol; and
(c) 0.1 to 0.9% (e.g. 0.2 to 0.8%, 0.3 to 0.7% such as 0.5%) w/w of a lubricant such as magnesium stearate.

In some embodiments, the ratio of mannitol:lubricant is preferably 10:1 or greater, preferably, 20:1 or greater e.g. 25:1 or greater such as 30:1 or greater (e.g. 30:1 to 35:1 such as 30.6:1 or 32.4:1).

In an embodiment, a solid composition for use in the invention does not include any added flavouring. In a preferred embodiment, a solid composition for use in the invention includes at least one flavouring. Suitable flavourings are available from various flavour manufacturers and suppliers, for example International Flavours and Fragrances Inc. (Duddery Hill, Haverhill, Suffolk, CB9 8LG, United Kingdom), Ungerer & Company (Sealand Road, Chester, CH1 4LP, United Kingdom), Firmenich (Firmenich UK Ltd., Hayes Road, Southall, Middlesex, UB2 5NN, United Kingdom) or S. Black Ltd (Foxholes Business Park, John Tate Road, Hertford, Herts, SG13 7YH, United Kingdom). Examples of suitable flavours include orange, lemon-lime, lemon, citrus, chocolate, tropical fruit, aloe vera, peppermint, tea, strawberry, grapefruit, blackcurrant, pineapple and vanilla, raspberry-lemon, cola flavour, and combinations thereof.

Preferred flavours are peppermint and raspberry-lemon flavour.

A flavouring may be integral in a solid composition, or it may be coated onto its surface. In one embodiment, the flavouring is integral in the solid composition. In such a solid composition, the flavouring preferably makes up 0.1 to 15% w/w of the solid composition. For example, the flavouring may make up 0.1 to 5% w/w of the solid composition, for example 0.1 to 2.0% w/w, for example 0.2 to 2.0% w/w. When the flavouring is peppermint, it is preferably present at a level of 0.1 to 1.0% w/w, for example 0.15 to 0.5% w/w. This level is particularly preferred when the solid of component (b) such as mannitol is present at a level of 14 to 17 % w/w of the solid composition of the invention. When the flavouring is raspberry-lemon, it is preferably present at a level of 0.5 to 2.0% w/w, for example 1.0 to 2.0%, for example 1.2 to 1.8% w/w. This level is particularly preferred when the solid of component (b) such as mannitol is present at a level of 14 to 17% w/w of the solid composition.

In an embodiment, the solid of component (b) such as mannitol and flavouring are, for example, present in a ratio of solid :flavouring of 170:1 to 3:1; when the flavouring is peppermint, the solid of component (b) such as mannitol and flavouring are preferably present in a ratio of solid :flavouring of 113:1 to 28:1. When the flavouring is raspberry-lemon, the solid of component (b) such as mannitol and flavouring are preferably present in a ratio of solid of component (b): flavouring of 14:1 to 7:1.

A solid composition for use in the invention may comprise one or more sweeteners. Sweeteners may be sugar-based. Preferably, they are not sugar-based. Preferred sweeteners include aspartame, acesulfame potassium (acesulfame K), sucralose and saccharine or combinations thereof. Alternatively, it can be preferred for the compositions to be substantially free from added sweeteners, for example to minimize the number of different components in the compositions. When present, sweeteners may, for example, be present in an amount of 0.01 to 1 % w/w. More preferably, a sweetener may be present in an amount of 0.1 to 1% w/w. The level of sweetener required to obtain a satisfactory taste may depend on the presence, and identity and quantity, of the other components of the composition.

In general it is not necessary for a solid composition for use in the invention to include preservatives or anti-oxidants. Nevertheless, low levels of anti-oxidants or preservatives may be included if required. It is preferred that compositions for use in the present invention are also substantially free from "salt taste" masking agents, such as agents that mask the taste of sodium sulphate, (other than flavourings mentioned herein) and from salts of non-fatty acids such as salts of mineral acids.

A solid composition for use in the invention can be of any convenient size. As mentioned above, a tablet should be sufficiently large to provide the desired quantity of PEG to the subject, but not be so large as to be uncomfortable in the mouth, difficult to chew or suck, or difficult to package. A tablet may, for example, have a mass of 0.5 to 10g, more preferably 0.5 to 5g, for example 1.0 to 5.0g, for example 2.0 to 3.5g, for example 2.5 to 3.5g. In one embodiment, a tablet for use in the invention has a mass of from 2.5 to 3.0g, for example 2.75g. For certain uses, where a larger amount of PEG is to be delivered to the subject, a larger tablet may be convenient, for example having a mass of 3 to 10g, for example 3 to 5g, 3 to 7g, 4 to 7g, or 5 to 8g, for example 4 to 7g. For certain uses, where a smaller amount of PEG is to be delivered to the subject, for example for paediatric uses, a smaller tablet may be convenient, for example having a mass of 0.5 to 2.0g, for example 1.0 to 1.75g, for example 1.25 to 1.50g.

A solid composition for use in the invention for preventing CRC may therefore be a solid composition of mass 2.0 to 3.5g comprising:
(a) 1.00 - 3.15g polyethylene glycol (PEG) having an average molecular weight within the range 2,000 to 10,000 Da; and
(b) 0.20 - 1.40g of the solid such as mannitol.

A solid composition for use in the invention for preventing CRC may therefore be a solid composition of mass 2.5 to 3.5g comprising:
(a) 1.25 - 3.15g polyethylene glycol (PEG) having an average molecular weight within the range 2,000 to 10,000 Da; and
(b) 0.25 - 1.40g of the solid such as mannitol.

Similarly, a composition for use in the invention for preventing CRC may therefore be a solid composition of mass 1.0 to 1.75g comprising:
(a) 0.50 - 1.575g polyethylene glycol (PEG) having an average molecular weight within the range 2,000 to 10,000 Da; and
(b) 0.10 - 0.70g of the solid such as mannitol.

As mentioned above, a lubricant (for example magnesium stearate) may be present in the solid composition in an amount of 2% w/w or less, for example 1% w/w or less. A solid composition for use in the invention of mass 2.0 to 3.5g may therefore comprise 0.07g or less of lubricant, for example 0.35g or less of lubricant. For example, it may comprise lubricant in an amount of 0.002 to 0.0315g, for example 0.004 to 0.028g, for example 0.006 to 0.0245g. A larger composition of mass 3.0 to 7.0g may comprise 0.14g or less of lubricant, for example 0.07g or less of lubricant. For example, it may comprise lubricant in an amount of 0.003 to 0.063g, for example 0.006 to 0.056g, for example 0.009 to 0.049g. A smaller composition of mass 1.0 to 1.75g may comprise 0.035g or less of lubricant, for example 0.0175g or less of lubricant. For example, it may comprise lubricant in an amount of 0.001 to 0.01575g, for example 0.002 to 0.014g, for example 0.003 to 0.01225g.

As mentioned above, flavouring may be present in the solid composition and, when present, it preferably makes up 0.1 to 15% w/w of the solid composition. A solid composition for use in the invention of mass 2.0 to 3.5g may therefore comprise 0.002 to 0.525g of flavouring, for example 0.002 to 0.175g, for example 0.002 to 0.07g, for example 0.004 to 0.07g of flavouring. A larger composition of mass 3.0 to 7.0g may comprise 0.003 to 1.05g of flavouring, for example 0.003 to 0.35g, for example 0.003 to 0.14g, for example 0.006 to 0.14g of flavouring. A smaller composition of mass 1.0 to 1.75g may comprise 0.001 to 0.2625g of flavouring, for example 0.001 to 0.0525g for example 0.001 to 0.021g, for example 0.002 to 0.021g of flavouring.

For example, a solid composition for use in the invention for preventing CRC may comprise:
(a) 50 - 90 % w/w polyethylene glycol (PEG) having an average molecular weight within the range 2,000 to 10,000 Da;
(b) 10 - 30 % w/w of the solid such as mannitol;
(c) 0.1 - 2.0 % w/w lubricant; and
(d) 0.1 - 15 % w/w flavouring.

In one embodiment, a solid composition for use in the invention for preventing CRC comprises:
(a) 70 - 90 % w/w polyethylene glycol (PEG) having an average molecular weight within the range 3,000 to 8,000 Da;
(b) 10 - 25 % w/w of the solid such as mannitol;
(c) 0.1 - 1.5 % w/w magnesium stearate; and
(d) 0.1 - 2.0 % w/w flavouring.

For example, a solid composition for use in the invention for preventing CRC comprises:
(a) 75 - 89 % w/w polyethylene glycol (PEG) having an average molecular weight within the range 3,000 to 4,000 Da;
(b) 10 - 20 % w/w of the solid such as mannitol;
(c) 0.2 - 0.8 % w/w magnesium stearate; and
(d) 0.1 - 1.0 % w/w flavouring.

For example, a solid composition for use in the invention for preventing CRC may comprise:
(a) 1.00 - 3.15g polyethylene glycol (PEG) having an average molecular weight within the range 2,000 to 10,000 Da;
(b) 0.20 - 1.40g of the solid such as mannitol;
(c) 0.002 - 0.07g lubricant; and
(d) 0.002 - 0.525g flavouring.

In one embodiment, a solid composition for use in the invention for preventing CRC comprises:
(a) 1.40 - 3.15g polyethylene glycol (PEG) having an average molecular weight within the range 3,000 to 8,000 Da;
(b) 0.20 - 0.875g of the solid such as mannitol;
(c) 0.002 - 0.0525g magnesium stearate; and
(d) 0.002 - 0.07g flavouring.

For example, a solid composition for use in the invention for preventing CRC comprises:
(a) 1.5 - 3.115g polyethylene glycol (PEG) having an average molecular weight within the range 3,000 to 4,000 Da;
(b) 0.20 - 0.70g of the solid such as mannitol;
(c) 0.004 - 0.016g magnesium stearate; and
(d) 0.002 - 0.035g flavouring.

For example, a solid composition for use in the invention for preventing CRC comprises:
(a) 2273 to 2284mg polyethylene glycol (PEG) having an average molecular weight within the range 3,000 to 4,000 Da;
(b) 420 to 446mg of the solid such as mannitol;
(c) 13.5 to 13.75mg magnesium stearate; and
(d) 11 to 42mg of flavouring such as peppermint or raspberry/lemon flavouring.

The solid compositions for use in the invention may be packaged in any convenient fashion. For example a plurality of units (e.g. tablets) of the solid composition (such as 5, 10, 15 or 20) may be packaged in a way conventional in the vitamin supplements industry. For example, they may be packed in a tube (such as a PTFE tube) equipped with a removable and replaceable closing means, for example a stopper. Alternatively, the solid compositions may be provided in a jar or other container with a removable and replaceable lid, or in a bag or within a wrapper (for example a foil wrapper). A desiccant is preferably also present. Alternatively, they may be packaged in a blister pack. In an embodiment, the solid compositions are packaged within a tube, jar, bag, wrapper or other container without any wrapping around individual units (e.g. tablets). Optionally, individual units of solid compositions may have a wrapping. In a preferred embodiment, 30 units of the composition are provided spilt into three tubes (e.g. 10 units per tube) or other packaging optionally together with instructions for use. Units may also be provided in a refill bag enabling previously obtained tubes to be refilled with units of the invention.

The solid compositions for use in the invention can be taken on their own as presented and chewed or sucked by a subject. It is not necessary for a subject to take water or another drink with the solid composition. Some subjects may wish to drink water or another fluid with or soon after taking a solid composition so as to facilitate the intake. The convenient packaging and the lack of a need to take water or another drink greatly increases the convenience of the solid compositions to subjects in comparison with other forms of PEG-based products currently on the market. Compositions for use in the present invention may be consumed prior to eating a meal or snack, together with the meal or snack or following a meal or snack.

In one embodiment, the subject typically takes up to 6g (or thereabout) per day of PEG, for example 2 to 6g per day, for example 3 to 5g per day, for example 4 to 5g per day. In that embodiment, the composition is free from components of a nature and quantity having a laxative effect. PEG is not considered to have significant laxative activity in an adult when taken at a level of 6g per day. Mannitol, flavouring and lubricant components are also not considered to have significant laxative activity at the daily levels at which they are provided when the composition provides up to 6g PEG per day. For a solid composition of 2.0 to 3.5g total mass and comprising 85% PEG w/w, a healthy subject may be recommended to take 1 or 2, or 1, 2 or 3 per day (to provide up to 6g or thereabout of PEG per day). For a smaller solid composition (of, for example, 1.0 to 1.75g total mass), a healthy subject may be recommended to take 1 to 6 per day, for example 2 to 5 per day (to provide up to 6g or thereabout of PEG per day). Conversely, for a larger solid composition (of, for example, total 3.0 to 7.0g total mass), a healthy subject may be recommended to take 1 or 2 per day (to provide up to 6g or thereabout of PEG per day).

Thus, the present invention provides a solid composition for oral administration as a solid (preferably having a mass of 1.0 to 5.0g) to a healthy subject (such as a human) for use in a method for preventing CRC and optionally preventing constipation as described *supra,*
wherein the composition comprises:
   (a) 50 - 90 % w/w (for example 60 to 90% w/w, preferably 70 to 90% w/w, more preferably 70 to 89 % w/w, for example 75 to 89 % w/w, e.g. 78 to 89 % w/w, 80 to 85% w/w, 81 to 85 % w/w, 80 to 84 % w/w, 82 to 84 % w/w) polyethylene glycol (PEG) having an average molecular weight within the range 2,000 to 10,000 Da; and
   (b) 10 - 40 % w/w (for example 10 to 25% w/w, 10 to 20% w/w, preferably 12 to 20 % w/w, more preferably 12 to 19% w/w, 12 to 18% w/w, or 12 to 17 % w/w, e.g.14 to 20% w/w, 14 to 19% w/w or 14 to 18% w/w 14 to 17% w/w) of a solid such as mannitol; together with
   (c) optional lubricant, optional flavouring and/or optional sweetener as described supra; and
wherein the method comprises administering said composition so that the subject consumes up to 6g (or thereabout) of PEG per day.

Preferably, said method comprises administering said composition so that the subject consumes between 2g and 6g, e.g. 2g to 5.5g per day. Preferably, said method is performed on a daily or alternate day basis. Preferably the method is performed over a period of at least two weeks, preferably at least a calendar month, more preferably at least 6 consecutive calendar months, or at least 12 consecutive calendar months, or at least 24 or at least 36 consecutive calendar months.

In certain embodiments of the invention described *supra,* the human subject may be one with a predisposition towards developing CRC based, e.g. on family history (e.g. family history of developing CRC), medical history (e.g. treatment of pre-existing colon polyps and/or the presence of a disease which predisposes an individual towards developing CRC e.g. familial polyposis or Lynch's syndrome and/or prior episode of CRC or colonic adenoma), health status or life style (for example due to high fat diet or heavy alcohol intake).

Since the present inventors have determined that the mean age for diagnosis of CRC in the dataset analysis given below is 73.0 years for males and 74.9 years for females, compositions of the invention may be used to prevent CRC in humans aged 50 years or greater, e.g. 55 years or greater such as 60 years or greater, e.g. 60 to 70 years or 65 to 70 or 60 to 75 years.

In other embodiments, the human subject is not constipated and the compositions for use in the present invention may both prevent constipation and prevent CRC in such humans.

Also disclosed herein is a kit (e.g. package) comprising a plurality of units (for example 5 or more, 10 or more, 20 or more) of compositions of the invention as described *supra* together with directions for use are provided. For example, the directions for use may state that one, two or three unit(s)/tablet(s) may be consumed per day either together or in a split dose (e.g. one tablet in the morning, one tablet in the evening). Accordingly therefore a composition in oral unit dosage form (e.g. tablet) for use in a method of preventing CRC (and, optionally, preventing constipation) in humans comprises an amount (e.g. effective and sub-laxative amount such as 2.2grams or thereabout) of PEG which method comprises administering one, two or three unit(s) of said composition per day. The composition may be used in a method such as described *supra.*

Compositions for use in the invention are therefore particularly beneficial for consumption by human subjects on a chronic (for example daily) basis since they have good mouthfeel and structural integrity and provide an amount of PEG useful in the context of colorectal cancer (particularly in conjunction with the methods of the invention) yet avoids the excessive gastrointestinal disturbances (such as loose stools) that may be regarded as unpleasant or generally unacceptable when consuming larger amounts of PEG on such a chronic basis.

It will be apparent to the reader of this specification, that the term "comprising" and grammatical variations thereof, in relation to embodiments of the invention described *supra,* may instead be "consisting essentially of' or "consisting of'.

The following examples illustrate but do not limit the invention.

### Example 1: Study of association between exposure to a macrogol laxative and the risk of colorectal cancer

### 1. Study objectives

- To describe the incidence of colorectal cancer on the General Practice Research Database (GPRD) following different types of laxative exposure.
- To evaluate any association between exposure to a macrogol laxative and the risk of colorectal cancer.

### 2. Methods

### Data source and study population

### The GPRD

The General Practice Research Database (GPRD) is a computerised database containing anonymised longitudinal data collected within UK primary care. It consists largely of coded data entered onto a computer system by general practitioners (GPs) as part of the clinical management of patients within primary care. Information entered onto the system includes demographic details, symptoms and medical diagnoses, detailed prescription data, hospital referrals and admissions and the results of clinical investigations and tests.

The longitudinal nature of the data combined with large sample sizes and high quality data on consultations, risk factors, diagnoses, prescribing and outcomes means the GPRD has a number of advantages for research. The age and sex distribution of the patient population in the database at any point in time closely matches the Office for National Statistics estimates for the total England and Wales population. In addition, for many co-morbidities, GPRD figures correlate well with those for other UK sources such as cancer statistics and figures reported by the Office for National Statistics. The GPRD is a widely used and validated source of epidemiological data and is a valuable resource for epidemiological research.

### Study design considerations

The nature of both the exposure and the outcome of interest for this study meant that the following considerations needed to be taken into account when deciding on the most appropriate study design:
1. Laxative users will differ in terms of their risk of CRC from non-laxative users. Therefore, to diminish the risk of 'confounding by indication' any association will need to be evaluated within the population of laxative users;
2. The risk of CRC increases exponentially with age. Therefore it is important that any matching between cases and controls is carried out based on the patients' year of birth and not on wider age bands;
3. CRC has a long latency period and exposure status at the time of diagnosis (classically the way exposure status is measured in epidemiological studies) will not be the relevant exposure status;
4. Changes in bowel movements might be the first presenting symptoms of CRC and therefore care needs to be taken when evaluating any association between laxative use and CRC that the laxatives were not prescribed because of the first presenting symptoms of CRC;
5. Laxative use is often on an 'as needed' basis, is intermittent, and people switch between products.

Given the complexities of the association of interest it was concluded that no single study design could provide the complete answer. In order to create a full picture of investigations, it was decided that it would be necessary to carry out a study of CRC incidence rates in different laxative exposure groups combined with a case-control study nested within the cohort study population.

The methods and results of both of these studies will now be discussed in turn.

### A. Descriptive studies of laxative use and CRC incidence

### Study period

Movicol® was licensed for use in the UK in 1998 and the study period ran from 1 January 2000 until 31 March 2009. Secular trends in prescribing data presented in this report, however, are given from January 1992 until December 2008: the last full calendar year for which data was available.

### Study population

The study population consisted of all patients on the GPRD permanently registered at, or transferred out of, a GP practice providing data that the MHRA considered to be up-to-standard for the purposes of research and who had received ≥ 1 laxative prescription during the time period their medical record was considered to be UTS (up-to-standard). There were no age restrictions.

### Identification of patients with colorectal cancer

Colorectal cancer was defined according to the International Classification of Diseases 9th edition and included ICD-9 153-154.1 (inclusive). The GPRD coding system, however, does not use ICD codes, so incident cases of colorectal cancer were identified on the GPRD using a combination of "read codes" and the application of three different algorithms.

### Identification of laxative users

Patients were eligible for inclusion in the cohort study from the day after their first recorded laxative prescription onwards. Laxative products were taken as those classified under chapter 1.6.1 - 1.6.5 of the British National Formulary (BNF). PEG based products at chapter 1.6.4 of the BNF include MOVICOL® which contains approximately 13.125g of macrogol 3350.

Patients were not included if their only laxative prescription(s) were recorded outside of the time period that their medical record was considered to be up-to-standard (UTS).

### Classification of laxative exposure

The laxative exposure of patients was classified into three categories
i. Non-macrogol user only
ii. Macrogol user only or before other laxative use
iii. Macrogol user after other laxative use

Up to the point someone received their first macrogol prescription on the GPRD, they were classified as 'non-macrogol user only'. From the point of receiving a prescription for a macrogol they were classified as either 'macrogol user only or before other' or 'macrogol user after other', as appropriate. This was done because, at the time of study, the following uncertainties existed: a) laxatives other than macrogols were not thought to cause any in- or decrease in CRC risk; b) it was unclear whether one dose of macrogol could have an impact on CRC risk, whether there was a threshold effect (a minimum number of dosages was needed to achieve a reduction in CRC risk) or whether there was a dose or duration-response association; and c) whether, if there was a reduction in CRC risk caused by macrogol utilisation, this was because of a reduction in tumour initiation or a reduction in tumour progression.

### B. Case-control study

### Study population

The case-control study was nested within the cohort of laxative users so all cases and controls were recruited from the laxative user cohort identified supra.

Cases and controls were eligible for study inclusion after the date of their first laxative prescription.

### Classification of laxative exposure

As with the cohort study, laxative exposure of patients was classified into three categories
i. Non-macrogol use only
ii. Macrogol use only or before other laxative use ('macrogol first')
iii. Macrogol use after other laxative use ('other laxative first')

Over the course of most of the study period, categories i. and iii. would have reflected common practice in accordance with prescribing guidelines, whereas category ii would have been unusual. This changed in 2006 and it is anticipated to change further following the recent publication of a Cochrane review that concludes macrogols are the laxative of first choice.

### Identification of colorectal cancer cases

Colorectal cancer cases were all patients with CRC newly diagnosed between 1 January 2000 and 31 March 2009 and they were identified using the algorithms and methods described for the cohort study supra. The index date was taken as the earliest date with evidence of a CRC diagnosis and was determined using the same criteria as for the descriptive study of CRC incidence rates. Colorectal cancer cases were required to have ≥6 months of UTS data before the CRC index date to enable information on covariates to be collected.

### Identification of controls

Six controls were identified for each CRC case in order to achieve optimum statistical power. By definition, controls have to be sampled from the population that gave rise to the cases. Therefore, controls were sampled from the same laxative cohort population and they were required to be disease free (have no diagnosis of colorectal cancer) on and before the index date of the case. In addition, to eliminate confounding by age and sex, they had to have the same year of birth and be of the same sex as the case.

Controls were also required to have ≥ 6 months of UTS data before the index date of the case to enable information on covariates to be collected and they were required to have received ≥1 prescription for a laxative before the CRC index date of the matched case.

### Power considerations

Based on the assumption macrogols had 10% of the laxative market share and with the selection of 6 controls per case, to demonstrate a 20% reduction in CRC risk the nested case-control study of CRC risk required approximately 2,360 newly diagnosed cases; to demonstrate a 25% reduction in risk 1,480 cases was required. If macrogols had 20% of the market then these numbers became 1,290 and 800 respectively.

### Back dating of the colorectal cancer index date

As exposure on the index date was unlikely to be relevant additional sets of controls were selected on the index date minus 6 months, minus 12 months, minus 18, 24, 30, 36, 42, 48, 54 and 60 months and for each of these analysis sets the analyses of exposure and risk factors were carried out based on the 'new' index dates. This means that any changes in exposure status or risk factors during the period between the 'new' index date and the original index date were ignored on the basis that these were irrelevant because the cancer was already present but had simply not been diagnosed yet.

It was hypothesised that if macrogol laxatives did decrease CRC risk then any association found should become stronger or remain stable with back dating of the index date.

### Dose response relationship

For the osmotic laxatives, the total dosage received (millilitres of lactulose and sachets of macrogols) was calculated for all cases and controls. The association between CRC risk and laxative dosage prescribed was evaluated.

Identification of data on potential risk factors for colorectal cancer

Information was collected, where available, for both cases and controls for each of the covariates listed below. CRC risk is related to diet and information on diet is not recorded within the GPRD. However, information on socioeconomic status (SES) and body mass index (BMI) was included as proxy measures.

Covariates present on the GPRD and reported or suggested to either be risk factors for, or have a protective effect against, colorectal cancer.

### Covariates

Smoking
Alcohol
Body mass index (BMI)
Socioeconomic status (SES)
Inflammatory bowel disease (IBD)
Cancer diagnosis other than colorectal cancer
Diabetes
Cholecystectomy
Prescriptions for:
   Low dose aspirin (<300mg)
   Non-low dose aspirin (≥300mg)
   COX-2 inhibitors
   Non-aspirin non-selective NSAIDs
   Dantron containing laxatives
   Statins
   Opioids
   Hormone replacement therapy (HRT)
   Calcium supplements
   5-ASA

### Statistical analyses

Conditional logistic regression analyses were carried out to evaluate any association between macrogol exposure and colorectal cancer risk. All covariates significant at the level of *p*<0.20 in the univariate analyses were considered for inclusion in the multivariate models. Covariates remained in the multivariate model if *p* ≤0.05 or if they altered the risk estimate by more than 10%. Tests were carried out for interactions between variables and the stability of the models was assessed using the Hosmer-Lemeshow statistic.

### C. Results

### Incidence of colorectal cancer on the GPRD.

A total of 14,598 potential incident CRC cases were identified on the GPRD between 1 January 2000 and 31 March 2009, in addition to which there were 255 cases of cancer of the anus that were included for the comparison of CRC incidence on the GPRD with UK figures reported by the cancer registries. Of the total 14,853 colorectal and anus cancer cases identified, 2,402 (16.7%) had a code specifically stating the type of cancer but with no record of supporting evidence for the diagnosis.

### Laxative drug utilisation on the GPRD

We identified 872,959 patients on the GPRD who had received a total of 9,866,699 laxative prescriptions between 1 January 1992 and 31 March 2009. A total of 721,513 macrogol laxative prescriptions were identified for 155,609 individuals, of which 1,297 (0.2%) were prescriptions for bowel cleansing preparations (Klean-Prep® or Moviprep®).

The annual number of laxative prescriptions recorded on the GPRD was found to increase over time in line with the increase in the number of patients contributing data to the database. In 2007 a total of 836,391 prescriptions were issued for laxative products. The reason for a slight reduction in the number of prescriptions recorded in 2008 is likely to be the result of the delay in some GP practices uploading their latest data collection to the MHRA. A continuous steady increase was observed in the number of prescriptions for macrogol laxatives following the first marketing authorisation of Movicol®.

Approximately two-thirds of laxative prescriptions on the GPRD were prescribed to females and one-third to males. A slightly larger proportion of macrogol prescriptions were issued to children and adolescents and to adults aged between 30 and 59 compared with all types of laxative prescriptions.

### Case-control study

We identified 4,734 eligible incident cases of colorectal cancer. Of the 4,734 CRC cases, 1,592 (33.6%) had only received a prescription for a laxative in the 6 months before the CRC index date.

Of these cases, 49.3% were male and 50.7% were female. CRC is more common in males but the slightly larger proportion of female cases identified is likely to reflect the age and sex distribution of the laxative user cohort combined with the fact that on average, women live longer than men. The mean age at diagnosis was 73.0 years (SD = 10.9) and 74.9 years (SD = 12.2) for males and females respectively.

These cases were therefore only eligible to be included in the index date analysis set - Table 1.

**Table 1: Number of cases and matched controls in each of the backdated analysis sets**

| Analysis set | Cases (n) | Controls (n) |
|---|---|---|
| Index date | 4,734 | 28,404 |
| Index date -6m | 3,142 | 18,852 |
| Index date -12m | 2,722 | 16,332 |
| Index date -18m | 2,445 | 14,670 |
| Index date -24m | 2,195 | 13,170 |
| Index date -30m | 1,982 | 11,892 |
| Index date -36m | 1,789 | 10,734 |
| Index date -42m | 1,636 | 9,816 |
| Index date -48m | 1,481 | 8,886 |
| Index date -54m | 1,351 | 8,106 |
| Index date -60m | 1,214 | 7,284 |

A potential dose-response relationship was observed for cohort in the "macrogol exposure following other" at 24 months (Table 2) and 36 months (Table 3) before the index date.

**Table 2 - Total Macrogol exposure 24 months before the index date.**

| No: of sachets | N | (Cases) | Odds Ratio | 95% CI |
|---|---|---|---|---|
| <20 | 99 | (14) | 1.02 | 0.57-1.80 |
| 21-30 | 124 | (17) | 0.98 | 0.58-1.66 |
| 31-60 | 154 | (22) | 0.97 | 0.62-1.54 |
| 61-180 | 144 | (13) | 0.57 | 0.32-1.02 |
| >180 | 140 | (11) | 0.50 | 0.27-0.94 |

**Table 3 - Total Macrogol exposure 36 months before the index date**

| No. of sachets | N | (cases) | Odds Ratio | 95% CI |
|---|---|---|---|---|
| <20 | 76 | (7) | 0.61 | 0.28-1.34 |
| 21-30 | 70 | (8) | 0.75 | 0.36-1.59 |
| 31-60 | 94 | (11) | 0.80 | 0.42-1.52 |
| 61-180 | 85 | (5) | 0.36 | 0.15-0.90 |
| >180 | 82 | (6) | 0.45 | 0.20-1.04 |

Since Movicol® contains approximately 13.125g of macrogol 3350 per sachet, a calculation can be made as to total macrogol exposure over the respective time frames that demonstrate a reduction in risk of CRC. See Tables 4 and 5 below wherein the total macrogol exposure over 24 months and 36 months respectively, together with 12 month, one month, one week and one day equivalent is provided.

**Table 4: 24 month Macrogol exposure (grams).**

| No: of sachets | 24 month | 12 month equivalent | One month equivalent | One week equivalent | One day equivalent |
|---|---|---|---|---|---|
| 61-180 | 800.6-2362.5 | 400.3-1181.3 | 33.3-98.4 | 7.7-22.7 | 1.1-3.2 |
| 180+ | 2362.5+ | 1181.3+ | 98.4+ | 22.7+ | 3.2+ |

**Table 5: 36 month Macrogol exposure (grams)**

| No: of sachets | 36 month | 12 month equivalent | One month equivalent | One week equivalent | One day equivalent |
|---|---|---|---|---|---|
| 61-180 | 800.6-2362.5 | 266.9-787.5 | 22.2-65.6 | 5.1-15.1 | 0.73-2.2 |
| 180+ | 2362.5+ | 787.5+ | 65.6+ | 15.1+ | 2.2+ |

### Example 2: Tablets containing different w/w % of mannitol, useful in the invention

The tablets described in Table 6 were prepared by combining the dry ingredients and compressing in a punch and die machine. For tablets 2A to 2C, the machine was a Manesty 16 punch D machine with a standard stainless steel punch and die with flat 22mm diameter and beveled edge with PTFE and vulcalon inserts from I Holland Ltd. For tablets 2D and 2E, the unit formula amounts were compressed on a Manesty D machine at normal manufacturing speed and with a standard stainless steel punch and die with flat 22mm diameter and beveled edge. The properties of the tablets were noted and they are given in Table 6b below.

Tablet 2A had an acceptable taste. However, the tablets were prone to sticking to the tableting machine, and many tablets were capped or laminated, making them unusable. Tablet 2B contained the same flavouring as tablet 2A, but more mannitol (15.3% vs. 9.1% in 2A) and less flavouring (1.5% vs. 5.4%). Tablet 2B had an acceptable taste and there was no evidence of sticking to the tableting machine, or capping or laminating of the tablets. Tablet 2C contains similar amounts of PEG, mannitol and magnesium stearate to tablet 2B, but the flavouring is peppermint. It displays similar characteristics to tablet 2B. Tablet 2D contained no flavouring, and 10% mannitol. It displayed no capping or sticking and only a small amount of chipping. Tablet 2E contained no flavouring, and 40% mannitol. It displayed good manufacturing characteristics. Tablets 2D and 2E had a bland taste as compared with tables 2A to 2C. This is most likely because of the absence of flavouring. The taste was, however, not unpleasant. Tablets 2F to 2H all displayed good manufacturing characteristics and an acceptable taste.

It is seen that a tablet containing from 59.5 to 89.5% w/w PEG (in particular 82.7 or 82.9% w/w PEG) and 10 to 40% /w mannitol (in particular 15.3 or 16.2% w/w mannitol) has better ease of manufacture characteristics than a tablet containing 85.0% PEG and 9.1% mannitol.

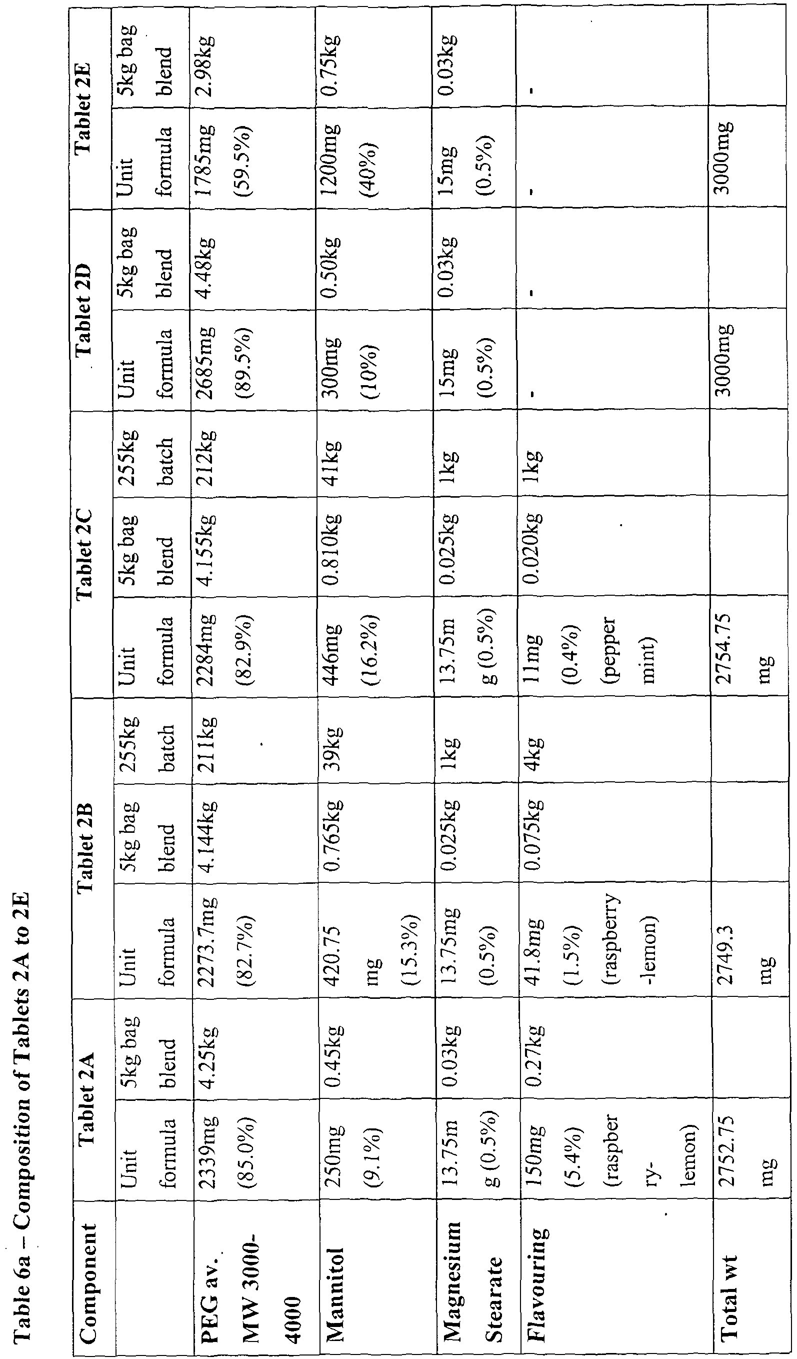

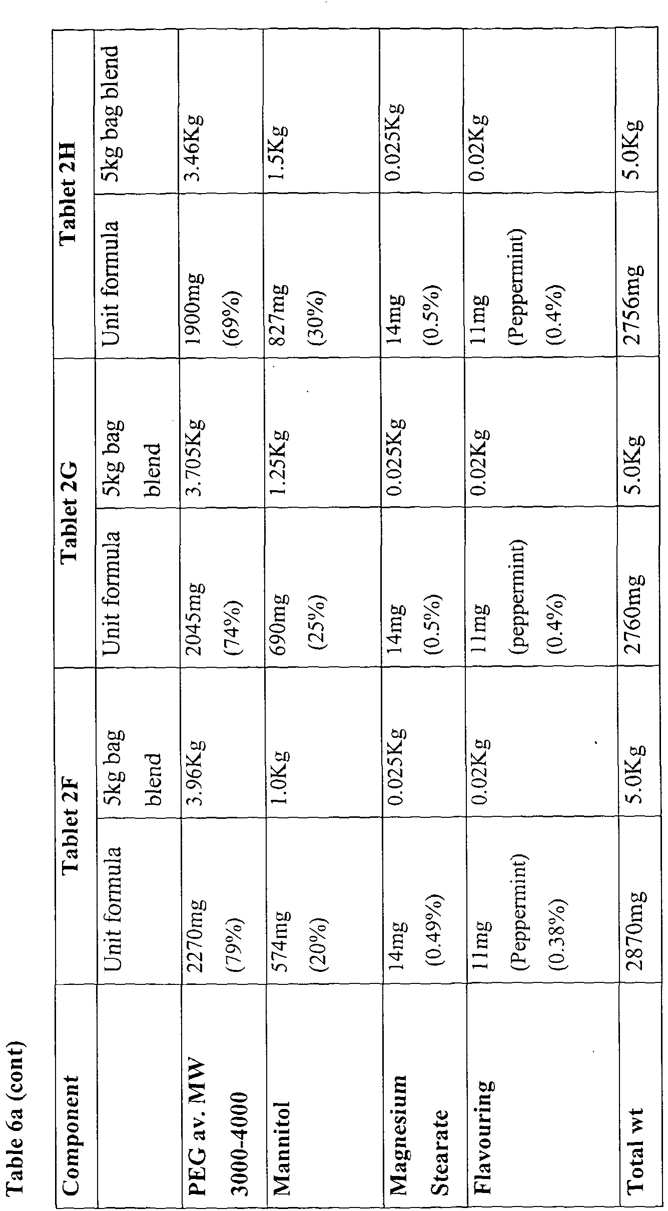

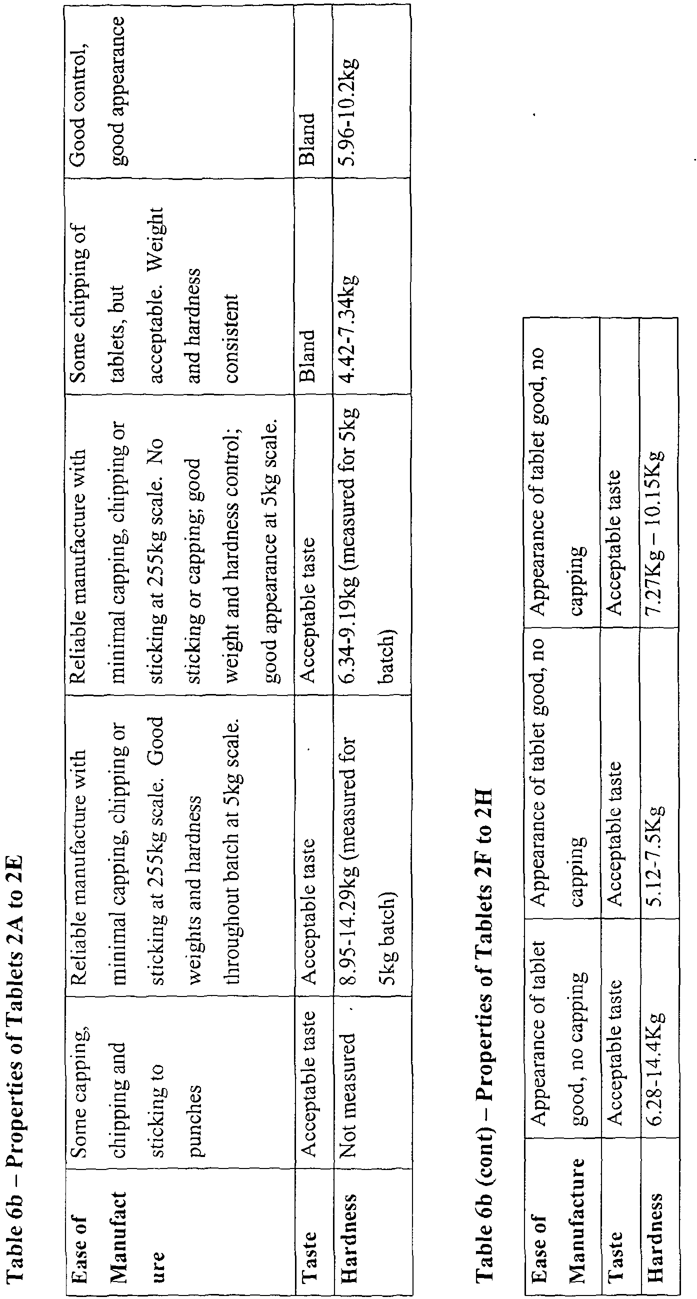

### Example 3: Comparison of tablets containing mannitol and different w/w % of magnesium stearate, useful in the invention

The tablets described in Table 7a were prepared. The materials were dispensed and then bag blended. The unit formula amounts were compressed on a Manesty D machine at normal manufacturing speed and with a standard stainless steel punch and die with flat 22mm diameter and beveled edge and PTFE inserts. The properties of the tablets were noted and they are given in Table 7b below.

It is seen in Tables 7a and 7b that tablets containing PEG and 15% w/w mannitol and 0.2%, 0.5% or 5.0% w/w magnesium stearate have good manufacturing properties and acceptable taste.

**Table 7a - Composition of Tablets 3A to 3C**

| **Component** | **Tablet 3A** | | **Tablet 3B** | | **Tablet 3C** | |
|---|---|---|---|---|---|---|
| | Unit formula | 5kg bag blend | Unit formula | 5kg bag blend | Unit formula | 5kg bag blend |
| **PEG average MW 3000-4000** | 2544mg (84.8%) | 4.24kg | 2400mg (80%) | 4.0kg | 2535mg (84.5%) | 4.23kg |
| **Mannitol** | 450mg (15%) | 0.75kg | 450 mg (15%) | 0.75kg | 450mg (15%) | 0.75kg |
| **Magnesium Stearate** | 6mg (0.2%) | 0.01kg | 150mg (5%) | 0.25kg | 15mg (0.5%) | 0.03kg |
| **Flavouring** | - | - | - | - | - | - |
| **Total wt** | 3000 mg | | 3000 mg | | 3000 mg | |

**Table 7b - Properties of Tablets 3A to 3C**

| | | | |
|---|---|---|---|
| **Ease of Manufacture** | Tablets good, no capping | Tablets good | Slight capping |
| **Taste** | Bland taste | Bland taste, slightly artificial | Better taste |
| **Hardness** | 5.4-11.0kg | 4.42-7.34kg | 0.4-6.1kg |

### Example 4: Comparison of various flavours of PEG+mannitol tablets, us the invention

Tablets analogous to the tablets of Example 2 with a variety of flavourings were prepared by combining the dry ingredients and compressing in a punch and die machine. The machine was a Manesty 16 punch D machine with a standard stainless steel punch and die with flat 22mm diameter and beveled edge with PTFE and vulcalon inserts from I Holland Ltd.

The tablets were provided to a panel of tasters. They were asked to taste each of the tablets and score them with an emphasis on the flavour, scoring from 1 (unpleasant) to 5 (pleasant). There were 22 tasters and their scores were summed together. It was found that peppermint flavoured tablets (score = 65) and lemon-raspberry flavoured tablets (score = 87) were preferred over lemon-lime flavoured tablets (score = 37) and orange flavoured tablets (score = 23).

### Example 5: Comparison of Tablets containing various other solids, useful in the invention.

Tablets containing solids other than mannitol were prepared in the same manner as the tablets of Example 2 *supra.* The composition and properties of these tablets are noted in Table 8a and 8b below.

**Table 8a - Comparison of Alternative Solids**

| **Component** | **Tablet 5A** | **Tablet 5B** | **Tablet 5C** |
|---|---|---|---|
| | Unit amount/ 5Kg blend | Unit amount/ 5Kg blend | Unit amount/ 5Kg blend |
| **PEG av. MW 3000-4000** | 2276mg/4.145Kg (83%) | 2279mg/4.145Kg (83%) | 2280mg/4.145Kg |
| **Solid** | Sorbitol | Lactose/Starch¹ | Xylitol |
| | 445mg/0.81Kg (16.2%) | 446mg/0.81Kg (16.2%) | 446mg/0.81Kg |
| **Magnesium Stearate** | 14mg/0.025Kg (0.51%) | 14mg/0.025Kg (0.51%) | 14mg/0.025Kg (0.51%) |
| **Flavouring** | 11 mg/0.02Kg (0.40%) | 11mg/0/02Kg (0.40%) | 11mg/0.02Kg (0.40%) |
| **Total wt** | 2746mg / 5.0Kg | 2753mg/5.0Kg | 2750mg/5.0Kg |

| | | | |
|---|---|---|---|
| 1. Lactose / starch compound, StarLac® (Roquette Pharma, Northants, spray-dried compound consisting of 85% alpha-lactose monohydrate (Ph. Eur. /USP-NF) and 15% maize starch (Ph. Eur. /USP-NF) dry matter. | | | |

**Table 8b - Properties of Tablets 5A to 5C**

| | | | |
|---|---|---|---|
| **Ease of Manufacture** | Tablet appearance good, no capping | Tablet appearance good, no capping | Tablet appearance good, no capping |
| **Taste** | Tasted ok, but lacked enhanced mouthfeel of mannitol tablets | Good hardness, tasted bland | Tasted ok but tablet too soft |
| **Hardness** | 6.97-10.09Kg | 4.13-9.4Kg | 4.67-7.99Kg |

### Example 6 - Comparison of Tablets containing further various solids, useful in the invention

Tablets containing solids other than mannitol were prepared in the same manner as the tablets of example 2 *supra.* The composition and properties of these tablets are noted in Table 9a and 9b below.

**Table 9a - Comparison of alternative solids**

| **Component** | **Tablet 6A** | **Tablet 6B** | **Tablet 6C** |
|---|---|---|---|
| | Unit amount/5Kg blend | Unit amount/5Kg blend | Unit amount/5Kg blend |
| **PEG av. MW 3000-4000** | 2279mg/4.145Kg (82.9%) | 2279mg/4.145Kg (82.9%) | 2263mg/4.145Kg (82.9%) |
| **Solid** | Lactose | Dextrate¹ | Cellulose² |
| | 445mg/0.810Kg (16.2%) | 440mg/0.800Kg (16.0%) | 442mg/0.810Kg (16.2%) |
| **Magnesium Stearate** | 14mg/0.025Kg (0.5%) | 14mg/0.025Kg (0.50%) | 13.6mg/0.025Kg (0.5%) |
| **Flavouring** | 11 mg/0.020Kg (0.4%) | 11 mg/0.020Kg (0.4%) | 11 mg/0.020Kg (0.4%) |
| **Total wt** | 2750mg/5.0Kg | 2746mg/4.99Kg | 2730mg/5.0Kg |

| | | | |
|---|---|---|---|
| ¹ Emdex®, available from JRS Pharma, Rosenberg, Germany. ² Avicel®, microcrystalline cellulose, available from FMC biopolymers, Philadelphia, USA. | | | |

**Table 9b - Properties of Tablets 6A to 6C**

| | | | |
|---|---|---|---|
| **Ease of Manufacture** | Tablet appearance good, no capping, hardness good | Tablet appearance good, some capping | Tablet appearance good, no capping |
| **Taste** | Taste ok, but very bland | Pleasant taste but quite soft | Taste unpleasant |
| **Hardness** | 5.2-13.6Kg | 5.4-11.9Kg | 7.1-13.4Kg |

## Claims

1. A composition for use in a method for preventing colorectal cancer (CRC) in humans, wherein the method comprises administering between 800 grams and 2365 grams of PEG or over a period of 18 to 36 consecutive calendar months.

2. The composition for use in a method for preventing CRC in humans of claim 1, wherein the administration is over a period of 24 consecutive calendar months or over a period of 36 consecutive calendar months.

3. A composition for use in a method for preventing colorectal cancer (CRC) in humans, wherein the method comprises administering between 0.1 grams and 6.0 grams of PEG on a daily basis.

4. The composition for use in a method for preventing CRC in humans of any preceding claim, for preventing CRC in humans and for the simultaneous use in preventing constipation and/or maintaining normal gastrointestinal transit time in humans.

5. The composition for use in a method for preventing CRC in humans of any preceding claim, wherein the method comprises administering between 266 and 1181 grams, for example between 400.3 and 787.5 grams, of PEG over a period of 12 consecutive calendar months.

6. The composition for use in a method for preventing CRC in humans of any preceding claim, wherein the method comprises administering between 22 and 98 grams of PEG over a period of one month.

7. The composition for use in a method for preventing CRC in humans of claim 1, wherein the method comprises administering between 5.1 and 22.7 grams of PEG over a period of one week.

8. The composition for use in a method for preventing CRC in humans of claim 1, wherein the method comprises administering between 0.73 and 3.22 grams of PEG over a period of one day.

9. The composition for use in a method for preventing CRC in humans of any preceding claim, comprising between 1.5 and 2.5 grams of PEG, for example between 2.0 and 2.5 grams.

10. The composition for use in a method for preventing CRC in humans of claim 9, which is administered daily or intermittently over a period of 24 or 36 consecutive calendar months.

11. The composition for use in a method for preventing CRC in humans of any preceding claim, wherein the PEG has an average molecular weight of between 1000 and 10,000 Daltons, for example between 3000 and 8000 Daltons, for example selected from the group consisting of 3350, 4000, 6000 and 8000 Daltons.

12. The composition for use in a method for preventing CRC in humans of any preceding claim, wherein the human is predisposed towards developing CRC (e.g. due to familial history and/or medical history such as a prior episode of CRC or colon polyps, Lynch's syndrome, familial polyposis and/or health status and/or lifestyle).

13. The composition for use in a method for preventing CRC in humans of any preceding claim, wherein the human is aged 50 years or greater, for example 55 years or greater, for example 60 years or greater, for example 60 to 75 years or 65 to 75 years.

14. The composition for use in a method for preventing CRC in humans of any preceding claim, wherein the composition is in oral dosage form.

15. The composition for use in a method for preventing CRC in humans of any preceding claim, wherein the composition is in the form of a tablet, capsule, caplet, troche, powder, granules, liquid.

16. The composition for use in a method for preventing CRC in humans of any preceding claim, wherein the composition comprises:
(a) 50 - 90 % w/w polyethylene glycol (PEG), having an average molecular weight within the range 2,000 to 10,000 Da; and
(b) 10 - 40 % w/w of a solid selected from the group: sorbitol, lactose, lactose and starch, dextrates, cellulose, xylitol, maltitol and mannitol;
(c) optionally further excipients such as flavourings, sweeteners and lubricants

17. The composition for use in a method for preventing CRC in humans according to claim 16, which is a solid composition for oral administration as a solid.

18. The composition for use in a method for preventing CRC in humans of claim 16 or claim 17, wherein the composition comprises:
(a) 70 - 90 % w/w polyethylene glycol (PEG) having an average molecular weight within the range 2,000 to 10,000 Da;
(b) 10 - 20 % w/w of the solid such as mannitol;
(c) 0 - 2.0% w/w lubricant; and
(d) 0 - 2.0% w/w flavouring.

19. The composition for use in a method for preventing CRC in humans of any one of claims 16 to 18, wherein the composition is substantially free from electrolytes.

20. The composition for use in a method for preventing CRC in humans as claimed in any one of claims 16 to 19, wherein the composition has a mass of 2.0 to 3.5g and comprises:
(a) 1.00 - 3.15g polyethylene glycol (PEG) having an average molecular weight within the range 2,000 to 10,000;
(b) 0.20 - 1.40g mannitol.

21. The composition for use in a method for preventing CRC in humans as claimed in claim 1, wherein the method comprises administering between 1.5 to 2.5 grams of PEG on a daily or intermittent basis, wherein approximately 800 grams or greater of PEG is administered over a period of 24 or 36 consecutive calendar months.

## Patentansprüche

1. Eine Zusammensetzung zur Verwendung bei einem Verfahren zur Prävention von Dickdarmkrebs (CRC) bei Menschen, wobei das Verfahren die Verabreichung zwischen 800 Gramm und 2365 Gramm PEG oder über einen Zeitraum von 18 bis 36 aufeinanderfolgenden Kalendermonaten umfasst.

2. Die Zusammensetzung zur Verwendung bei einem Verfahren zur Prävention von CRC bei Menschen nach Anspruch 1, wobei die Verabreichung über einen Zeitraum von 24 aufeinanderfolgenden Kalendermonaten oder über einen Zeitraum von 36 aufeinanderfolgenden Kalendermonaten erfolgt.

3. Eine Zusammensetzung zur Verwendung bei einem Verfahren zur Prävention von Dickdarmkrebs (CRC) bei Menschen, wobei das Verfahren die Verabreichung zwischen 0,1 Gramm und 6,0 Gramm PEG täglich umfasst.

4. Die Zusammensetzung zur Verwendung bei einem Verfahren zur Prävention von CRC bei Menschen nach einem vorhergehenden Anspruch zur Prävention von CRC bei Menschen und zur gleichzeitigen Verwendung zur Prävention von Obstipation und/oder Aufrechterhaltung einer normalen gastrointestinalen Transitzeit bei Menschen.

5. Die Zusammensetzung zur Verwendung bei einem Verfahren zur Prävention von CRC bei Menschen nach einem vorhergehenden Anspruch, wobei das Verfahren die Verabreichung zwischen 266 und 1181 Gramm, zum Beispiel zwischen 400,3 und 787,5 Gramm, PEG über einen Zeitraum von 12 aufeinanderfolgenden Kalendermonaten umfasst.

6. Die Zusammensetzung zur Verwendung bei einem Verfahren zur Prävention von CRC bei Menschen nach einem vorhergehenden Anspruch, wobei das Verfahren die Verabreichung zwischen 22 und 98 Gramm PEG über einen Zeitraum von einem Monat umfasst.

7. Die Zusammensetzung zur Verwendung bei einem Verfahren zur Prävention von CRC bei Menschen nach Anspruch 1, wobei das Verfahren die Verabreichung zwischen 5,1 und 22,7 Gramm PEG über einen Zeitraum von einer Woche umfasst.

8. Die Zusammensetzung zur Verwendung bei einem Verfahren zur Prävention von CRC bei Menschen nach Anspruch 1, wobei das Verfahren die Verabreichung zwischen 0,73 und 3,22 Gramm PEG über einen Zeitraum von einem Tag umfasst.

9. Die Zusammensetzung zur Verwendung bei einem Verfahren zur Prävention von CRC bei Menschen nach einem vorhergehenden Anspruch, umfassend zwischen 1,5 und 2,5 Gramm PEG, zum Beispiel zwischen 2,0 und 2,5 Gramm.

10. Die Zusammensetzung zur Verwendung bei einem Verfahren zur Prävention von CRC bei Menschen nach Anspruch 9, die täglich oder intermittierend über einen Zeitraum von 24 oder 36 aufeinanderfolgenden Kalendermonaten verabreicht wird.

11. Die Zusammensetzung zur Verwendung bei einem Verfahren zur Prävention von CRC bei Menschen nach einem vorhergehenden Anspruch, wobei das PEG eine mittlere Molekülmasse von zwischen 1000 und 10000 Dalton besitzt, zum Beispiel zwischen 3000 und 8000 Dalton, zum Beispiel ausgewählt aus der Gruppe bestehend aus 3350, 4000, 6000 und 8000 Dalton.

12. Die Zusammensetzung zur Verwendung bei einem Verfahren zur Prävention von CRC bei Menschen nach einem vorhergehenden Anspruch, wobei der Mensch prädisponiert ist für die Ausbildung von CRC (z.B. aufgrund familiärer Vorbelastung und/oder Anamnese wie z.B. einer früheren CRC-Episode oder Dickdarmpolypen, Lynch-Syndrom, familiärer Polyposis und/oder Gesundheitszustand und/oder Lebensgewohnheiten).

13. Die Zusammensetzung zur Verwendung bei einem Verfahren zur Prävention von CRC bei Menschen nach einem vorhergehenden Anspruch, wobei der Mensch 50 Jahre oder älter ist, zum Beispiel 55 Jahre oder älter, zum Beispiel 60 Jahre oder älter, zum Beispiel 60 bis 75 Jahre oder 65 bis 75 Jahre.

14. Die Zusammensetzung zur Verwendung bei einem Verfahren zur Prävention von CRC bei Menschen nach einem vorhergehenden Anspruch, wobei die Zusammensetzung in oraler Darreichungsform vorliegt.

15. Die Zusammensetzung zur Verwendung bei einem Verfahren zur Prävention von CRC bei Menschen nach einem vorhergehenden Anspruch, wobei die Zusammensetzung in Form einer Tablette, Kapsel, kapselförmigen Tablette, Pastille, eines Pulvers, Granulats oder einer Flüssigkeit vorliegt.

16. Die Zusammensetzung zur Verwendung bei einem Verfahren zur Prävention von CRC bei Menschen nach einem vorhergehenden Anspruch, wobei die Zusammensetzung umfasst:
(a) 50 - 90% Gew./Gew. Polyethylenglycol (PEG) mit einer mittleren Molekülmasse im Bereich von 2000 bis 10000 Da, und
(b) 10 - 40% Gew./Gew. eines Feststoffs, ausgewählt aus der Gruppe: Sorbit, Lactose, Lactose und Stärke, Dextrate, Cellulose, Xylit, Maltit und Mannit,
(c) gegebenenfalls weitere Hilfsstoffe wie z.B. Aromastoffe, Süßstoffe und Gleitmittel.

17. Die Zusammensetzung zur Verwendung bei einem Verfahren zur Prävention von CRC bei Menschen gemäß Anspruch 16, die eine feste Zusammensetzung zur oralen Verabreichung als Feststoff ist.

18. Die Zusammensetzung zur Verwendung bei einem Verfahren zur Prävention von CRC bei Menschen nach Anspruch 16 oder Anspruch 17, wobei die Zusammensetzung umfasst:
(a) 70 - 90% Gew./Gew. Polyethylenglycol (PEG) mit einer mittleren Molekülmasse im Bereich von 2000 bis 10000 Da,
(b) 10 - 20% Gew./Gew. des Feststoffs wie z.B. Mannit,
(c) 0 - 2,0% Gew./Gew. Gleitmittel und
(d) 0 - 2,0% Gew./Gew. Aromastoff.

19. Die Zusammensetzung zur Verwendung bei einem Verfahren zur Prävention von CRC bei Menschen nach einem der Ansprüche 16 bis 18, wobei die Zusammensetzung im Wesentlichen frei von Elektrolyten ist.

20. Die Zusammensetzung zur Verwendung bei einem Verfahren zur Prävention von CRC bei Menschen wie in einem der Ansprüche 16 bis 19 beansprucht, wobei die Zusammensetzung eine Masse von 2,0 bis 3,5 g besitzt und umfasst:
(a) 1,00 - 3,15 g Polyethylenglycol (PEG) mit einer mittleren Molekülmasse im Bereich von 2000 bis 10000 Da,
(b) 0,20 - 1,40 g Mannit.

21. Die Zusammensetzung zur Verwendung bei einem Verfahren zur Prävention von CRC bei Menschen wie in Anspruch 1 beansprucht, wobei das Verfahren die Verabreichung zwischen 1,5 bis 2,5 Gramm PEG täglich oder intermittierend umfasst, wobei etwa 800 Gramm oder mehr PEG über einen Zeitraum von 24 bis 36 aufeinanderfolgenden Kalendermonaten verabreicht werden.

## Revendications

1. Une composition destinée à être utilisée dans un procédé de prévention du cancer colorectal (CRC) chez l'homme, dans laquelle le procédé comprend l'administration de 800 grammes à 2365 grammes de PEG ou pendant une période de 18 à 36 mois civils consécutifs.

2. La composition à utiliser dans un procédé de prévention du CRC chez l'homme selon la revendication 1, dans laquelle l'administration se déroule sur une période de 24 mois civils consécutifs ou sur une période de 36 mois civils consécutifs.

3. La composition à utiliser dans une méthode de prévention du cancer colorectal (CRC) chez l'homme, dans laquelle la méthode comprend l'administration quotidienne de 0,1 gramme à 6,0 grammes de PEG.

4. La composition à utiliser dans un procédé de prévention du CRC chez l'homme selon l'une quelconque des revendications précédentes, pour prévenir le CRC chez l'homme et pour l'utilisation simultanée dans la prévention de la constipation et/ou le maintien d'un temps de transit gastro-intestinal normal chez l'homme.

5. La composition à utiliser dans un procédé de prévention du CRC chez l'homme selon l'une quelconque des revendications précédentes, dans laquelle le procédé comprend l'administration d'entre 266 et 1181 grammes, par exemple entre 400,3 et 787,5 grammes, de PEG sur une période de 12 mois civils consécutifs.

6. La composition à utiliser dans un procédé de prévention du CRC chez l'homme selon l'une quelconque des revendications précédentes, dans laquelle le procédé comprend l'administration entre 22 et 98 grammes de PEG sur une période d'un mois.

7. La composition à utiliser dans un procédé de prévention du CRC chez l'homme selon la revendication 1, dans laquelle le procédé comprend l'administration d'entre 5,1 et 22,7 grammes de PEG sur une période d'une semaine.

8. La composition à utiliser dans un procédé de prévention du CRC chez l'homme selon la revendication 1, dans laquelle le procédé comprend l'administration d'entre 0,73 et 3,22 grammes de PEG sur une période d'un jour.

9. La composition à utiliser dans un procédé de prévention du CRC chez l'homme selon l'une quelconque des revendications précédentes, comprenant entre 1,5 et 2,5 grammes de PEG, par exemple entre 2,0 et 2,5 grammes.

10. La composition à utiliser dans une méthode de prévention du CRC chez l'homme selon la revendication 9, qui est administrée quotidiennement ou par intermittence sur une période de 24 ou 36 mois civils consécutifs.

11. La composition à utiliser dans un procédé de prévention du CRC chez l'homme selon l'une quelconque des revendications précédentes, dans laquelle le PEG a un poids moléculaire moyen compris entre 1000 et 10000 Daltons, par exemple entre 3000 et 8000 Daltons, par exemple choisi dans le groupe constitué de 3350, 4000, 6000 et 8000 Daltons.

12. La composition à utiliser dans un procédé de prévention du CRC chez l'homme selon l'une quelconque des revendications précédentes, lorsque l'homme est prédisposé à développer un CRC (par exemple en raison d'antécédents familiaux et/ou d'antécédents médicaux tels qu'un épisode antérieur de CRC ou de polypes du côlon, syndrome de Lynch, polypose familiale et/ou état de santé et/ou mode de vie).

13. La composition à utiliser dans un procédé de prévention du CRC chez l'homme selon l'une quelconque des revendications précédentes, lorsque l'humain est âgé de 50 ans ou plus, par exemple 55 ans ou plus, par exemple 60 ans ou plus, par exemple 60 à 75 ans ou 65 à 75 ans.

14. La composition à utiliser dans un procédé de prévention du CRC chez l'homme selon l'une quelconque des revendications précédentes, dans laquelle la composition est sous forme posologique orale.

15. La composition à utiliser dans un procédé de prévention du CRC chez l'homme selon l'une quelconque des revendications précédentes, dans laquelle la composition est sous la forme d'un comprimé, d'une capsule, d'un cachet, d'une pastille (troche), d'une poudre, de granulés, d'un liquide.

16. La composition à utiliser dans un procédé de prévention du CRC chez l'homme selon l'une quelconque des revendications précédentes, dans laquelle la composition comprend:
(a) 50 à 90% en poids/poids de polyéthylène glycol (PEG), ayant un poids moléculaire moyen compris entre 2 000 et 10 000 Da; et
(b) 40% en poids/poids d'un solide choisi dans le groupe: sorbitol, lactose, lactose et amidon, dextrates, cellulose, xylitol, maltitol et mannitol;
(c) éventuellement d'autres excipients tels que des arômes, des édulcorants et des lubrifiants

17. La composition à utiliser dans un procédé pour prévenir le CRC chez l'homme selon la revendication 16, qui est une composition solide pour une administration orale sous forme solide.

18. La composition à utiliser dans un procédé de prévention du CRC chez l'homme selon la revendication 16 ou la revendication 17, dans laquelle la composition comprend:
(a) 70 à 90% en poids/poids de polyéthylène glycol (PEG) ayant un poids moléculaire moyen compris entre 2 000 et 10 000 Da;
(b) 10 à 20% en poids/poids du solide tel que le mannitol;
(c) 0 - 2,0% en poids/poids de lubrifiant; et
(d) 0 à 2,0% en poids/poids d'arôme.

19. La composition à utiliser dans un procédé de prévention du CRC chez l'homme selon l'une quelconque des revendications 16 à 18, dans laquelle la composition est sensiblement exempte d'électrolytes.

20. La composition destinée à être utilisée dans un procédé de prévention du CRC chez l'homme selon l'une quelconque des revendications 16 à 19, dans laquelle la composition a une masse de 2,0 à 3,5 g et comprend:
(a) 1,00 - 3,15 g de polyéthylène glycol (PEG) ayant un poids moléculaire moyen compris entre 2 000 et 10 000;
(b) 0,20 - 1,40 g de mannitol.

21. La composition destinée à être utilisée dans un procédé de prévention du CRC chez l'homme selon la revendication 1, dans lequel le procédé comprend l'administration de 1,5 à 2,5 grammes de PEG sur une base quotidienne ou intermittente, dans lequel environ 800 grammes ou plus de PEG sont administrés sur une période de 24 ou 36 mois civils consécutifs.
